# EUROPEAN PATENT APPLICATION

(11) **EP 0 726 240 A1**
(43) Date of publication of application: **14.08.1996**
(21) Application number: 96500019.3
(22) Date of filing: 07.02.1996
(51) Int. Cl.: C05F 11/00, C05F 11/10, C12P 13/00

(54) **Obtaining and use of diamines, polyamines and other complementary active elements from treated natural products**

(30) Priority: 10.02.1995 ES 9500274
(71) Applicant: TAGUIDELL, S.L., 25210 Guissona (Lleida) (ES)
(72) Inventor: Salavert Casamor, Agusti, E-08208 Sabadell (Barcelona) (ES)
(74) Representative: Aragones Forner, Rafael Angel

(57) **Abstract**

SUMMARY

The present invention refers in first instance the knowledge of the last generation of fertilizers with very low conductivity and containing polyamines (cadaverine and putrescine, among others). The synergism between cadaverine and putrescine allows a significant reduction of the concentration of N.P.K. contained in the "conventional" fertilizers.

The invention also comprises the method of obtaining fertilizers from specific industrial waste products or subproducts with difficult ecological reuse. Its application leads to a significant reduction of the harvesting periods and a great increase of the yield of different crops.

## Description

The aim of the present invention is the obtaining of nutritional products, based on diamines, polyamines and other complementary active elements, from treated natural products, of major proteic composition, its use and application in plants, animals and humans.

In the present invention, by treated natural products it is included all products of natural origin of major proteic composition, that they had have different kind of treatments, state changes, high changes of temperature, and/or pH, fermentations, digestions, biological actions, long incubations, varied processes of proteolysis and another treatments that denature, degrade or permit the break of covalent bounds of proteins and peptides, making easier to obtain the constituent amino acids. These treated natural products also contain fermented natural products and/or digested material considered as unvalued waste products or collateral subproducts which are difficult to reuse and its ecological elimination is highly costly.

The present invention describes the obtaining of diamines, (between them cadaverine and putrescine), and other polyamines and another complementary active elements, and its application as a manure or fertilizer. The present invention describes the method of obtaining peptides and amino acids by aerobic and anaerobic incubations of proteins coming from natural products, as described above.

The present invention has also the aim of using and applying the mentioned products as fertilizers, manure, substrate, bionutrient, biostimulatory, bioactivator, corrector, emendation or additive, as well as their possible applications in nutrition (animal and human nourishment), perfumery and cosmetics (aromas).

The present invention describes that the synergistic effect of cadaverine and putrescine allows a significant reduction of salt concentration (N.P.K) normally used in conventional manures or fertilizers. This has an important ecological impact since it reduces the "classical" problem of soil salinity through the continuous and massive use of nitrogen fertilizers.

The use of the products described in the present invention produces a delay of plant senescence, a reduction of the harvest time, and significant increases of crop productivity.

The diamines (putrescine and cadaverine, among others) were first identified in animal tissues subjected to bacterial decomposition and putrefaction, being this the origin of the peculiar denomination of these compounds. In the beginning, it was thought that they were toxic compounds coming from the putrefaction of organic material, and that they were responsible of the toxicity of the poisonous food. In that time these compounds were named as "ptomamines" (from greek "ptoma" or corps). However, the idea about the toxic nature of these compounds radically changed from Herbs and Snell discovery (Putrescine as growth factor for *Hemophylus parainfluenzae.* J. Biol. Chem. 176:989,1948), who discovered that putrescine was a growth factor for *Hemophylus parainfluenzae* bacteria. So, in the last paragraph of their work, these authors indicated "that it was the first demonstration of the essential nutritive function of one of the putrefaction-amines".

Subsequently, many studies have demonstrated that some polyamines are essential growth factors not only in bacteria and animals but also in plants. The diamine putrescine is widely distributed in the plant kingdom. In contrast, the diamine cadaverine is much less distributed than putrescine; its effects and function are much less studied and its synergistic effect with putrescine has never been described. See review in Tiburcio, AF, Kaur-Sawhney, R and Galston AW (Polyamine metabolism. In The Biochemistry of Plants, Intermediary Nitrogen Fixation, BJ Miflin and PJ Lea, (eds.), Vol 16, New York: Academic Press, Inc., pp. 283-325, 1990). The application of exogenous polyamines as well as the manipulation of their endogenous levels produces effects in all stages of plant development (see review in Tiburcio et al. 1990).

The described diamines are:
- Putrescine: 1,4 Diaminobutane or Tetramethylendiamine.
   Formula:

   NH₂-CH₂-CH₂-CH₂-CH₂-NH₂
- Cadaverine: 1,5 Diaminopentane or 1,5 Pentanediamine or Pentamethylendiamine
   Formula:

   NH₂-CH₂-CH₂-CH₂-CH₂-CH₂-NH₂

From the literature, it has been performed a revision in order to know the current use or application of the diamines (putrescine and cadaverine) and other polyamines, as manure or fertilizer, based on "VADEMECUM 1995 of Fitosanitary and Nutritional Products" by Carlos De Lifian, 11a Edition. I.S.B.N. 84-87480-05-5 Ediciones AGROTECNICAS, S.L. It is clear that these diamines are not mentioned in neither of these formulated products, and no reference or use of these active elements exists in the different compositions and formulations of all the products cited in the VADEMECUM-1995. Neither there is a mention of its applications in either tables and index of such VADEMECUM. Therefore, this indicates that there is not mention of its use, and its commercial application for the moment.

The patent JP-B-47008588 of Ajimoto Inc. describes the existence of microorganisms capable to produce 1,3-diaminopropane, but no method for obtaining other diamines and polyamines, and other complementary active compounds is mentioned. The aim of this invention is the use of 1,3-diaminopropane as antiviral agent.

The aim of the present invention is the obtaining of diamines coming from treated natural products of proteic composition. As primary material it uses natural products of major proteic composition subjected to partial or total degradation which have already been used in specific industrial processes and considered in most cases as waste products or subproducts of difficult application or reuse. So that confers to the present invention an unheard of and additional ecological interest which is completely different from the aims of the Ajimoto patent. The 3rd Claim of the present invention describes the obtaining of the diamines putrescine and cadaverine (1,4-diaminobutane and 1,5-diaminopentane, respectively), which is not aimed in the Ajimoto patent. The present invention describes the properties of the diamines and active elements obtained with this method as efficient mediators of plant growth and/or as regulatory factors with wide positive action. This permits its application as fertilizers, manure, substrate, bionutrient, biostimulatory, bioactivator, corrector, emendation or additive for any already existing related product. These characteristics are also not mentioned in the Ajimoto patent.

The patent JP-A-06256105 of Suntory Ltd. describes that at least one diamine which general formula including 2 and 9 atoms of carbon or their salts induces the flowering in certain conditions (long day conditions). In certain conditions it is able to artificially control the plant flowering; although it is only concentrated in just one example of plant: morning glory *(Pharbitis nil* cv. Kidachi). It exclusively emphasizes the flower induction and describes a very concrete methodology to achieve the control of flowering in a determinated case only related to the gardening field; never other knowledge, neither application in horticulture, cereals, fruits and other applications related to nourishment production (animal and human), nor the improvement of crop productivity is mentioned in the Suntory patent. More importantly, this patent do not mention the possible synergistic relations of putrescine ad cadaverine.

The present invention, in claims 1,2 and 3, describes a method for obtaining or manufacturing diamines, polyamines and other complementary elements, using as a raw materials natural products of major proteic composition, coming from industrial processes, which contain fermented natural products and/or digested material considered as unvalued waste products which are difficult to reuse. All of this confers an additional and unheard of ecological interest which is completely different from the aims of the Suntory patent.

The present invention, in the claims 5,6 and 8, emphasizes the sinergism between cadaverine and putrescine, including their different applications and origins, which permits a significant reduction of the saline concentration (N.P.K.) in relation to the "classical" concept of manure or fertilizer. The substitution of these salts by putrescine and cadaverine leads to equal and/or improved practical results. Furthermore, the combination of putrescine and cadaverine gives better results than the application of these diamines alone. All these effects fit with the concept of sinergism defined by Salisbury and Ross 1991 (Plant Physiology 4th Ed. pp. 562) as: "If two factors interact in such a way that the response to them given together is greater than the sum of responses to each given alone, we speak of sinergism".

The Suntory patent emphasizes exclusively the flowering induction, but, in contrast to the present invention, does not mention in its results the regulation of growth, a general antisenescence effect in the vegetative organs of the plants, the increase of size and quality of leaves, flowers and fruits, reduction of harvest time, increase of aromatic intensity, large number of fruits and seeds and therefore a great improvement of industrial productivity. Furthermore, the present invention describes as well the application as fertilizer or manure in the conditions mentioned in claim 10 or as a general fertilizer or manure, or used as additive, substrate, bionutrient, biostimulatory, bioactivator, corrector or emendation; all of this is not mentioned in the Suntory patent. Additionally, the present invention enphasizes the decrease of the N.P.K concentrations to limits even lower that those established in the "law of the minimum" (Salisbury and Ross. 1991.Plant Physiology 4th Ed. pp. 552-574).

There is a publication by Gamarnik and Frydman (Chemical Abstracts vol. 115; Plant Physiol. vol. 97:778-785 en 1991) entitled "Cadaverine, an essential diamine for the normal root development of germinating soybean seeds" that reveals the possible function of cadaverine in the development of soybean roots. Furthermore, in this work other polyamines such as spermidine and spermine, not specifically mentioned in the present invention, have been studied. The present invention, in claim 4, describes the using of cadaverine as additive in fertilizers and manures in general, which is not mentioned in the work of Gamarnik and Frydman, just concentrated on the role of cadaverine in soybean root development. At the end of this publication it is stated that there is too much to clarify about the function of the diamine cadaverine. The present invention, in claim 8, describes a manure which uses the sinergism between cadaverine and putrescine as well as its application and use as a general fertilizer. This objective is also not mentioned in the Gamarnik and Frydman' s work.

There is the publication by Rodriguez Jerez et al. (Chemical Abstracts vol. 122; J. Food Sci. vol. 50, 998-1001 en 1994) entitled "Histamine, cadaverine and putrescine forming bacteria from ripened Spanish semipreserved anchovies" that describes the possible toxicity of semipreserved anchovies and studies the presence of histamine and other amines such as putrescine and cadaverine. Although these diamines are studied, the method of obtaining cadaverine and putrescine and its possible application as fertilizers or manures is not mentioned in contrast to the claims 1 and 3 of the present invention.

The patent EP-A-0193904 of Fujisawa Pharmaceutical develops and details the process and methods for obtaining amine compounds related to the following formulations: 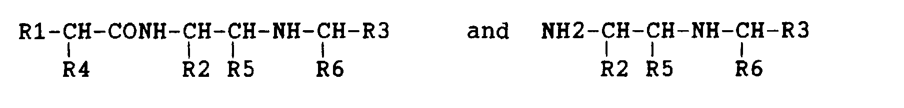 wherein:
- R1: is hydrogen, amino or a protected amino group,
- R2: is hydrogen, carboxyl or a protected carboxy group,
- R3: is hydrogen, carboxyl or a protected carboxy group,
- R4: is lower alkyl, amino(lower)alkyl, protected amino (lower)alkyl, carbamoyl(lower)alkyl or protected carbamoyl(lower)alkyl,
- R5: is hydrogen or lower alkyl, and
- R6: is hydrogen, or lower alkyl, or heterocyclic alkyl,
These formulations do not include in any case the diamines putrescine and cadaverine obtained according to the present invention. Moreover, it claims qualities and pharmacological properties for these compounds that in any case are close to the claims of the present invention which enphasizes the use of cadaverine and putrescine as fertilizers or manures.

The present invention describes a method of obtaining of diamines(putrescine and cadaverine among them), other polyamines and another active elements from treated natural products, of major proteic composition, by a process of aerobic and/or anaerobic incubations of proteins, peptides and amino acids, coming from treated natural products, in combination with other components.The method of the present invention includes the following stages:
a) Identification of the origin of the treated natural products (as described above) , rejectionfo the non-desirable ones, study and combination of the concentrations of considered essential compounds and acceptable complementary ones, and mixing and homogenization of them.
b) aerobic incubation: the essential and complementary compounds are subjected to inoculation orsowing of adequate microorganisms to growth inaerobiosis (to facilitate, contribute and participate in the decarboxylation of specific amino acids) , protease addition, homogenization of the mixture and aireation of the process. A control of the process is effectuated which includes the identification and quantification of the bacterial flora, and determinationof the concentrations of the main active elements (putrescine and cadaverine among others), with control of pH and temperature.
c) Anaerobic incubation: The suspension obtained by aerobic incubation is homogenized and a new inoculation or sowing of adequate microorganisms to growth inaerobiosis to facilitate, contribute and participate in the decarboxylation of specific amino acids is performed.
   The culture is again homogenized, and is placed in hermetic tanks without air. At the end of the process, the bacterial flora is inactivated, the concentrations of the main active elements (putrescine and cadaverine among others) are determined, with final control of pH.
d) Dosage and final packaging of the product.

According to the invention, the resulting final product,before packaging, is subjected to a process to obtain the diamines putrescine and cadaverine.

The present invention comprises the use of the diamines putrescine and cadaverine, obtained according to the present invention, as additives in fertilizers and manures.

Moreover, the present invention comprises the use of the diamine cadaverine, irrespective of its obtaining or origin, as additive in fertilizers and manures. The present invention also comprises the use of the diamines putrescine and cadaverine together, irrespective of their obtaining or origins, as additives in fertilizers and manures, in which cadaverine promotes the action of putrescine,or vice versa (sinergism).

The present invention comprises a manure and/or fertilizer which includes among other active components the diamine cadaverine.

Moreover, the present invention comprises of a manure and/or fertilizer that includes among other active components a combination of the diamines putrescine and cadaverine, in which cadaverine improves the action of putrescine, or vice versa (sinergism).

### DETAILED DESCRIPTION OF THE METHOD OF INVENTION

Previous stage include:
A) The identification of the origin of the treated natural products.
B) Rejection of treated natural products containing non-desirable chemical compounds such as metallic toxics : mercury (Hg), chromium (Cr), arsenic (As), Cadmium (Cd), Selenium (Se) and Lead (Pb), especially, and inhibitors of cell growth; pesticides; antibiotics; detergents and soaps; fats and oils; cyanides and fenolics or any other inhibitor of cell growth, in toxic concentrations for its development.
C) Study concentrations of considered essential components and of complementary components.
D) Combination and adjustment of concentration of selected components in order to achieve their right concentrations.
E) Mixing and homogenization of different treated natural products.

### Aerobic incubation:

A) Conditions:
   - Temperature of 5 to 60°C
   - pH between 2 and 10
   - Inoculation or sowing of the adequate microorganisms to grow in anaerobiosis to facilitate, contribute and participate in the decarboxylation of specific amino acids.
   - Addition of enzymes with proteolytic activity.
   - Homogenization of mixture and aireation of process.
   - Length of time variable according to the origin and composition of selected products.
B) Essential components:
   - Total brute protein superior to 1 mg/ml.
   - Free amino acids superior to 1% (on dry weight basis).
   - Total nitrogen superior to 0.1%.
   - Phosphorus (expressed as phosphoric anhydre, P₂O₅) superior to 0.1%, (1 g/l).
   - potassium (expressed as potassium anhydre K₂O) superior to 0.1%, (1 g/l).
   - Soluble sulphates (SO₄=) superior to 0.1% (1 g/l)
   - Sodium (expressed as sodium oxide, Na2O) superior to 0.1% (1 g/l).
   - Calcium (expressed as calcium oxide, CaO) superior to 0.1% (1 g/l).
   - Magnesium (expressed as magnesium oxide, MgO) superior to 0.1% (1 g/l).
C) Complementary components:
   - Iron (Fe) superior to 0.001 mg/l.
   - Manganese (Mn) superior to 0.0005 mg/l.
      - Bore (Bo) superior to 0.0005 mg/l.
      - Zinc (Zn) superior to 0.0001 mg/l.
   - Copper (Cu) superior to 0.0001 mg/l.
   - Cobalt (Co) superior to 0.0001 mg/l.
   - Molibdene (Mo) superior to 0.0001 mg/l.
D) Control of the Process:
   - Identification and quantification of bacterial flora.
   - Determination of concentration of the main active elements, putrescine and cadaverine among others.
   - Control of pH and temperature of the process.
   Once the aerobic incubation is finished the following incubation is performed:
   Anaerobic incubation:
   A volume of the suspension obtained in the aerobic incubation, previously homogenized, is subjected to the following conditions:
A)
   - Temperature of 5 to 60°C
   - pH between 2 and 10
   - Inoculation or sowing of the adequate microorganisms to grow in aerobiosis to facilitate, contribute and participate in the decarboxylation of specific amino acids.
   - Addition of enzymes with proteolytic activity.
   - Homogenization of mixture and storage in hermetically closed tanks.
   - Length of time variable according to the origin and composition of selected products.
B) Once the anaerobic incubation is finished, the obtained final suspension is homogenized and the following controls are carried out:
   - Identification and quantification of the bacterial flora
   - Partial or total inactivation of specific microorganisms
   - Determination of the concentrations of the main active elements putrescine and cadaverine among others.
   - Final control of pH.
C) The packaging of previously filtered final product is performed in closed bottle.

In the composition of the manure or fertilizer described in the present invention, the sinergism between cadaverine and putrescine leads to the achievement of a significant reduction of N.P.K. concentrations thus avoiding an excessive soil salinization; being its composition as follows:
A) Main elements:
   - Total brute protein superior to 1 mg/ml.
   - Free amino acids superior to 1 % (on dry weight basis).
   - Diamines putrescine and cadaverine in concentration superior to 0.1 mg/l of each.
   - Total nitrogen superior to 0.1%.
   - Phosphorus (expressed as phosphoric anhydre P₂O₅ superior to 0.1%, (1 g/l).
   - potassium (expressed as potassium anhydre K₂O) superior to 0.1%, (1 g/l).
B) Secondary elements:
   - Soluble sulphates (SO₄=) superior to 0.1% (lg/l).
   - Sodium (expressed as sodium oxide, Na2O) superior to 0.1% (1g/l).
   - Calcium (expressed as calcium oxide, CaO) superior to 0.1% (1g/l).
   - Magnesium (expressed as magnesium oxide, MgO) superior to 0.1% (1g/l)
C) Oligoelements:
   - Iron (Fe) superior to 0.001 mg/l.
   - Manganese (Mn) superior to 0.0005 mg/l.
   - Bore (Bo) superior to 0.0005 mg/l.
   - Zinc (Zn) superior to 0.0001 mg/l.
   - Copper (Cu) superior to 0.0001 mg/l.
   - Cobalt (Co) superior to 0.0001 mg/l.
   - Molibdene (Mo) superior to 0.0001 mg/l.

It should be mentioned that the conventional fertilizers contain a minimum concentration of N.P.K. (Nitrogen, Phosphorus, Potassium) oscillating between 6 to 45%. A concentration of N.P.K. lower than 6% of the total is not considered as a fertilizer in some Legislations. The present invention allows to significantly reduce the saline concentrations (N.P.K.) below the 6%, while obtaining improved practical results.

### EXPERIMENTAL RESULTS OBTAINED

### I) EXPERIMENTAL RESULTS FROM LABORATORY STUDIES

### A) In Avena saliva (important cereal of interest in human and animal nutrition):

The fertilizing solution of the invention has been the object of a study in which the influence of different dilutions of this solution on the growth of *Avena sativa* cv. Victory plants grown on vermiculite (inert substrate) has been investigated. The obtained results show a great antisenescence properties during vegetative growth, a stimulation of cell elongation, significant advancement of flowering period, and significant reduction of fructification and harvest time. The sinergism of cadaverine and putrescine has been demonstrated using this experimental system. Thus, the combination of cadaverine and putrescine were able to induce flowering and fruit set; on the contrary, individual treatments of cadaverine and putrescine did not induce flowering. B) In soybean (plant with interest in human and animal nutrition):

The fertilizer of the present invention has been investigated to study the influence on the development of soybean plants grown on vermiculite. Treatment with the fertilizer of the invention leads to normal flowering and fruit set of the plants, contrarily to the results obtained with other commercially available fertilizers which were unable to induce flowers and fruits in the soybean plants.

### II) EXPERIMENTAL RESULTS FROM GARDENING STUDIES

### A) Dracaena deremensis (Indoor ornamental plant).

The obtained results indicate that the plants treated with the fertilizer of the present invention showed leaves with larger size (between 50-70% higher than the controls). Similar effects were observed in duplicate experiments placing the plants in different locations. Moreover, the treated plants showed a generous flowering and the flowers presented a very strong aroma as compared with controls.

### B) In grass (application in gardening and sport-fields):

The obtained results with the use of the fertilizer of the present invention showed an improvement of the root system: increase of root ramification, increased diameter of main roots and improved root elongation. Technical quantification of the results showed a significant increase of the biological fresh and dry weight mass.

### III) EXPERIMENTAL RESULTS FROM FIELD STUDIES (HORTICULTURE):

### A) In tomato (Solanum licopersicum):

The results obtained with the use of the fertilizer of the present invention were as follows:
- There was a difference of one month of advancement of the harvest time between the treated plants and the control ones.
- The final yield obtained, measured in Kg of fruit harvested per plant, was improved by more than a 50% in the treated plants in relation to the controls.

### B) In bean plants (using two different commercial varieties):

- It was observed a significant difference in the degree of flower abscision produced by adverse environmental conditions between the treated plants and the controls.
- The bean plants treated with the fertilizer of the invention showed an advancement of the vegetative and reproductive growth leading to a significant advancement of harvest period and a great increase of yield per plant.

### C) In pepper plants:

- It was observed a significant reduction by the treatment of the water stress and chlorosis of plants, typical symptoms induced during plant transplantation from the seedbed (nursery) to the field.
- The size of the treated plants was significantly superior than the control plants.
- The treated plants showed a significant advancement of the flowering and fruit set periods.

### D) In endives:

- Two months after sowing, the treated plants showed a vegetative growth size of 3 to 4-fold higher than the controls.

Other significant positive results have been observed in the growth of other plants, obtained either in laboratory experiments as well as in field studies. The application of the fertilizer of the present invention shows positive experimental results in the gardening field ( common geranium, ferns, Potus, Diplodenias, *Phalaenopsis, Cadleyas..);* in horticulture (eggplant, pea, broadbean, potato,..); in fruticulture (strawberry, pear, plum,..); and model laboratory plants such as *Arabidopsis thaliana.*

### SUMMARY OF THE RESULTS:

The obtained results demonstrate that the active elements contained in the manure or fertilizer of the present invention positively participate in all the phases of plant development. The application of the cited active elements are manifested in the following points:
a) Reduction of the negative effects (water stress and chlorosis) induced during transplantation from the seedbed to the field.
b) Regulation of the plant growth.
c) Antisenescence effects in the vegetative organs.
d) Reduction of the flowering time
e) Increased resistance to flower abscision produced by adverse environmental conditions.
f) Increase of the aroma intensity of the flowers.
g) Improved size and quality of fruits.
h) Reduction of harvest-period.

In general, it shows a marked antisenescence effect of the vegetative plant growth leading to a growth stimulus and a great increase in the plant yield.

The application of the obtained results allow an improved and more effective control of the plant growth leading to an improved productivity with direct repercussions in gardening, horticulture, fruticulture and agriculture in general. It is also expected to show direct and positive consequences in human and animal nutrition; and repercussions in the quality of aromas with applications in perfumery and cosmetics.

The cited results regarding the diamines (putrescine and cadaverine), polyamines and other complementary active elements, and the manure or fertilizer of the present invention reveal the following:
- The fertilizer of the present invention acts significantly promoting the growth of ornamental and food plants; also showing antisenescence effects (delay of aging), reducing the period of vegetative growth and improving the yield of most of the crops tested.
- By quantitative analysis it has been verified that this fertilizer contains the diamines putrescine and cadaverine in enough concentration to produce physiological effects on the plant. Moreover, it allows (without showing any negative effect for the plant) a significant reduction of the N.P.K. concentrations which has an important ecological impact since prevents the excessive accumulation of salts in the soil destined for plant culture.
- The physiological effects of the cited fertilizer are superior to those described for putrescine by itself. It should be pointed out that very little is known about the effects of cadaverine by itself, and its essential importance for the plant cell growth has not been established until the present invention. For the first time, the present invention reveals the combined effect of cadaverine and putrescine, in which cadaverine improves the effect of putrescine, or vice versa (sinergism).

D) The method described in the present invention, uses as primary material products of natural origin of major proteic composition, partial or totally degraded, already used in specific industrial processes and considered as unvalued waste products or collateral subproducts which are difficult to reuse and its ecological elimination is highly costly. All of this confers to the described method an unheard of and additional ecological value.

It should be understood that in the practical realization of the method of invention to obtain diamines (putrescine and cadaverine, among them), other polyamines and another complementary active elements, and the manure and/or fertilizer, as well as its use and application, some variations could be produced without altering the essential characteristics.

## Claims

1. Method of obtaining of diamines, polyamines and other complementary active elements from treated natural products, of major proteic-composition, which is characterized by including a process of aerobic and/or anaerobic incubations of proteins, peptides and amino acids coming from treated natural products, as it has been defined in the invention.

2. Method, according to claim 1, characterized by the following phases:
a) Previous stage: Include the identification of the origin of the treated natural products, rejection of the non-desirable ones, study and combination of the concentrations of considered essential compounds and acceptable complementary ones, and mixing and homogenization of them.
b) aerobic incubation: the essential and complementary compounds are subjected to inoculation or sowing of adequate microorganisms to growth in aerobiosis (to facilitate, contribute and/or participate in the decarboxylation of specific amino acids), protease addition, homogenization of the mixture and aireation of the process, and a control of the process is effectuated which includes the identification and quantification of the bacterial flora, determination of the concentrations of the main active elements (putrescine and cadaverine, mainly), with control of pH and temperature of the process.
c) Anaerobic incubation: The suspension obtained by aerobic incubation is homogenized; then is subjected to inoculation or sowing of adequate microorganisms to growth in anaerobiosis (to facilitate, contribute and/or participate in the decarboxylation of specific amino acids); the culture is again homogenized, and is placed in hermetic tanks.
d) Final control of the process: identification and quantification of the bacterial flora is performed, determination of the concentrations of the main active elements (putrescine and cadaverine mainly), with final control of pH.

3. Method, according to claims 1 and 2, characterized by the resultant final product which is subjected to a process of separation for obtaining the diamines putrescine and cadaverine.

4. Use of diamine cadaverine as additive in fertilizers and manures.

5. Use of diamines putrescine and cadaverine together as additives in fertilizers and manures, in which cadaverine is improving the putrescine action, or vice versa (synergism).

6. Use of diamines putrescine and cadaverine according to claims 1, 2 and 3, characterized by the use and application of these diamines as additives in fertilizers and manures.

7. Manure, including among other active compounds the diamine cadaverine.

8. Manure, including among other active compounds the diamines putrescine and cadaverine together, in which cadaverine improves the putrescine action, or vice versa (synergism).

9. Manure, according to claims 1, 2 and 3, comprising among other active compounds the diamines putrescine and cadaverine obtained by aerobic and/or anaerobic incubations of proteins, peptides and amino acids coming from treated natural products, as described above.

10. Manure, characterized by presenting a low concentration of N.P.K., as main elements, thus avoiding the accumulation of salts in the soil, and substituting them by the incorporation of cadaverine and putrescine (synergism); with the following composition:
A) Main elements:
- Total brute protein superior to 1 mg/ml.
- Free amino acids superior to 1 % (on dry weight basis).
- Diamines putrescine and cadaverine in concentration superior to 0.1 mg/l of each.
- Total nitrogen superior to 0.1%.
- Phosphorus (expressed as phosphoric anhydre P₂O₅ superior to 0.1%, (1 g/l).
- potassium (expressed as potassium anhydre K₂O) superior to 0.1%, (1 g/l).
B) Secondary elements:
- Soluble sulphates (SO₄=) superior to 0.1% (1g/l).
- Sodium (expressed as sodium oxide, Na₂O) superior to 0.1% (1g/l).
- Calcium (expressed as calcium oxide, CaO) superior to 0.1% (1g/l).
- Magnesium (expressed as magnesium oxide, MgO) superior to 0.1% (1g/l)
C) Oligoelements:
- Iron (Fe) superior to 0.001 mg/l.
- Manganese (Mn) superior to 0.0005 mg/l.
- Bore (Bo) superior to 0.0005 mg/l.
- Zinc (Zn) superior to 0.0001 mg/l.
- Copper (Cu) superior to 0.0001 mg/l.
- Cobalt (Co) superior to 0.0001 mg/l.
- Molibdene (Mo) superior to 0.0001 mg/l.
